# EUROPEAN PATENT APPLICATION

(11) **EP 4 212 190 A2**
(43) Date of publication of application: **19.07.2023**
(21) Application number: 23150064.6
(22) Date of filing: 02.01.2023
(51) Int. Cl.: A61L 9/20

(54) **SURFACE DISINFECTION AND VISIBLE LIGHT SYSTEM AND METHOD FOR USING SAME**

(30) Priority: 12.01.2022 US 202263298696 P; 11.04.2022 US 202217717722
(71) Applicant: Safran Cabin Germany GmbH, 35745 Herborn (DE); Safran Cabin Inc., Huntington Beach, CA 92647 (US)
(72) Inventor: TOMASENA, Etienne, 77550 MOISSY-CRAMAYEL (FR); HOLLM, Marco, 77550 MOISSY-CRAMAYEL (FR); MECKES, Ruediger, 77550 MOISSY-CRAMAYEL (FR); MATTAR, Sherif, 77550 MOISSY-CRAMAYEL (FR); SMITHSON, Tyler, 77550 MOISSY-CRAMAYEL (FR); DE LA CRUZ, Marie, 77550 MOISSY-CRAMAYEL (FR)
(74) Representative: Gevers & Orès

(57) **Abstract**

Surface disinfection for enclosures with Ultra-Violet (UV) light and illuminating such enclosures with visible light. Particular embodiments relate to limiting the transmission of viruses and bacteria in the interior space of an aircraft, such as a lavatory, through the use of UV light application, while providing visible light to passengers of such lavatory, both in a safe and compliant manner. This disclosure provides a lighting system and method that combines a visible light emitter and a UV light emitter into a single light system module. Although UV light is a preferred method of neutralizing pathogens, its use is not safe for human exposure. Accordingly, the disclosed lighting system is capable of detecting the presence of an occupant within an enclosure or defined space and only allows the visible light emitter to function (and prevents the UV light emitter from functioning) while people or animals are present within the electromagnetic range for the lighting system. The lighting system can then detect the absence of an occupant within an enclosure or defined space, prior to activating the UV light emitter, and once absence is detected, the UV light emitter can be activated by a controller.

## Description

### CROSS REFERENCE TO RELATED APPLICATIONS

This application is related to and claims priority benefits from U.S. Provisional Application Serial No. 63/298,696, filed on January 12, 2022, entitled "SURFACE DISINFECTION ND VISIBLE LIGHT SYSTEM AND METHOD FOR USING SAME," the entire contents of which are hereby incorporated in its entirety by this reference.

### FIELD OF THE DISCLOSURE

The field of this disclosure relates generally to disinfecting enclosures with Ultra-Violet (UV) light and illuminating such enclosures with visible light. Particular embodiments relate to limiting the transmission of viruses and bacteria in the interior space of an aircraft, such as a lavatory, through the use of UV light application, while providing visible light to passengers of such lavatory, both in a safe and compliant manner. This disclosure provides a lighting system and method that combines a visible light emitter and a UV light emitter into a single light system module. Although UV light is a preferred method of neutralizing pathogens, its use is not safe for human exposure. Accordingly, the disclosed lighting system is capable of detecting the presence of an occupant within an enclosure or defined space and only allows the visible light emitter to function (and prevents the UV light emitter from functioning) while people or animals are present within the electromagnetic range for the lighting system. The lighting system can then detect the absence of an occupant within an enclosure or defined space, prior to activating the UV light emitter, and once absence is detected, the UV light emitter can be activated by a controller.

### BACKGROUND

Commercial airline operations are being disrupted by the COVID 19 crisis. Airline passenger trips have reduced significantly, causing a financial crisis in the industry. Airlines are interested in developing and deploying technology, products, and solutions that will clean, sanitize, disinfect, or even sterilize the aircraft interior before, during, or after flight. Some solutions include wiping surfaces with cleaning liquids, replacing the materials used in high-touch surface areas with materials that have anti-microbial properties, adding films to high touch surfaces with anti-microbial properties or using Ultra-Violet (UV) lights as pathogen killing devices.

Although many concepts are being developed and proposed to airline operators that kill pathogens, such as the COVID 19 corona virus, there is still a need to ensure these technologies, products, or solutions also meet the industry needs of economy, efficiency and safety. Moreover, commercial products are being adapted for use on the aircraft, but these products may not meet the industry requirements to allow them to fly or to be certified as airworthy. To be certified as airworthy, equipment installed on the aircraft, including but not limited to enclosures, must meet all applicable requirements, referred to as "airworthiness regulations," of the certifying airworthiness authority, for example, the Federal Aviation Administration (FAA) in the United States and the European Union Aviation Safety Agency (EASA) in Europe.

Companies design, manufacture, certify and sell commercial aircraft enclosures. Examples of such enclosures include, but are not limited to, lavatories, galleys, closets, crew rests, and overhead stowage bins. In order to lessen the transmission of viruses and bacteria, collectively referred to as pathogens, such enclosures may be disinfected through the use of UV light that will reduce the transmission of pathogens between passengers traveling on commercial aircraft. Where such enclosures are already on an aircraft and are certified under applicable airworthiness regulations, replacement or refurbishment enclosures must also meet all such applicable airworthiness regulations and be economical to install.

The current state of the art for surface sanitization is cleaning liquids and UVC technology. Cleaning liquids and other anti-microbial materials are cost effective and relatively safe for humans but need a long residual time to efficiently neutralize pathogens. UVC technology can efficiently and quickly neutralize all type of bacteria and viruses, however human exposure is considered to be carcinogenic. Thus, both of these disinfection methods have limitations.

The current state of the art for visible light on aircraft or in enclosed spaces, such as an aircraft lavatory, is Red Green Blue Light Emitting Diodes, commonly known as RGB LED. By adjusting the brightness of each of the three red, green and blue LEDs, it is possible to adjust the color and the intensity of the visible light in the enclosure, such as inside an aircraft lavatory. Such lighting systems already exist in aircraft lavatories, making the addition of a second, independent UV lighting system space consuming, heavy and more expensive. Improvements are thus desirable.

### SUMMARY

The terms "disclosure," "invention," "the invention," "this invention" and "the present invention" used in this patent are intended to refer broadly to all of the subject matter of this patent and the patent claims below. Statements containing these terms should be understood not to limit the subject matter described herein or to limit the meaning or scope of the patent claims below. Embodiments of the invention covered by this patent are defined by the claims below, not this summary. This summary is a high-level overview of various aspects of the invention and introduces some of the concepts that are further described in the Detailed Description section below. This summary is not intended to identify key or essential features of the claimed subject matter, nor is it intended to be used in isolation to determine the scope of the claimed subject matter. The subject matter should be understood by reference to appropriate portions of the entire specification of this patent, any or all drawings and each claim.

Currently aircraft enclosures provide interior lighting in the visible spectrum. To disinfect the inside of an enclosure or interior monument, UV lighting may be used to kill pathogens, such as bacteria or viruses. UV light may only be used when no humans or animals are present in the enclosure. Typically, any UV light disinfection system is provided as a completely separate system. In order to reduce cost and weight, while simultaneously ensuring passenger safety, the present disclosure combines these two lighting systems (visible spectrum lighting and UV disinfection lighting) into a single system, while complying with all applicable airworthiness regulations. One or more detectors and/or sensors are provided that ensure that the UV light emitter is only activated when there is not an occupant in the enclosure. The one or more detectors and/or sensors send their detected/sensed information to a controller, and the controller activates or deactivates the different light emitters.

According to certain embodiments of the present invention, there is provided a lighting system module that contains both UV light emitters and visible light emitters. The system may be configured for installation in an enclosure to be disinfected and may include a light system module that has at least one UV light emitter and at least one visible light emitter, wherein the at least one UV light emitter is anti-microbial in nature, anti-viral in nature, or both, wherein the visible light emitter emits light in the visible spectrum; one or more detectors or sensors, the one or more detectors or sensors comprising (a) a presence detector that detects the presence of people or animals within the enclosure and/or within an electromagnetic range of the at least one UV light emitter, (b) a door locked/unlocked sensor, (c) a door open/closed sensor, or (d) any combination thereof; and a controller that manages the light system module and the one or more detectors or sensors.

The present invention proposes therefore a lighting system comprising a light system module, wherein such light system module contains both UV light emitters and visible light emitters.

The present invention further proposes a lighting system for installation in an enclosure to be disinfected, the lighting system comprising:
a light system module that has at least one UV light emitter and at least one visible light emitter, wherein the at least one UV light emitter is anti-microbial in nature, anti-viral in nature, or both, wherein the visible light emitter emits light in the visible spectrum;
one or more detectors or sensors, the one or more detectors or sensors comprising (a) a presence detector that detects the presence of people or animals within the enclosure and/or within an electromagnetic range of the at least one UV light emitter, (b) a door locked/unlocked sensor, (c) a door open/closed sensor, or (d) any combination thereof; and
a controller that manages the light system module and the one or more detectors or sensors.

The present invention may comprise one or more of the following features, considered alone or in combination with one another:
- the UV light emitter is anti-microbial in nature, anti-viral in nature, or both;
- the lighting system is used or installed within an enclosure;
- the lighting system further comprises one or more of:
   one or more presence detectors that detect the presence of people or animals within an enclosure and/or within the electromagnetic range of the UV light emitter;
   a door locked/unlocked sensor;
   a door open/closed sensor;
   or any combination thereof;
- the lighting system further comprises a controller that manages the light system module, the one or more presence detectors, the door locked/unlocked sensor, and/or the door open/closed sensor;
- the lighting system is certified by the relevant airworthiness authority to be compliant with applicable airworthiness regulations;
- the lighting system is configured to be installed within the fuselage of an aircraft;
- the lighting system is configured to be installed within an interior of a train, boat, bus, or other transportation vehicle;
- the lighting system is configured to be installed in a lavatory of a hotel, a stadium, a movie theater, or other enclosed public place;
- the one or more detectors or sensors are configured to detect the presence or absence of an occupant in the enclosure and send detected information to the controller, wherein the controller can activate the at least one UV light emitter if the detected information relays absence of an occupant, wherein the controller can activate the at least one visible light emitter if the detected information relays presence of an occupant.

The present invention further proposes an aircraft fuselage, or an interior of a train, boat, bus, or other transportation vehicle, or a lavatory of a hotel, a stadium, a movie theater, or other enclosed public place, wherein a lighting system as described above is installed.

The present invention further proposes a method for using a lighting system, such as the lighting system as described above, to neutralize pathogens and provide visible light within the interior of an enclosure, the method comprising:
(a) detecting that there are no people or animals within the enclosure,
(b) detecting that the enclosure door is closed,
(c) detecting that the enclosure door is unlocked,
(d) neutralizing pathogens within the enclosure by switching on a UV light emitter when a controller has determined the disinfection operational mode can be activated,
(e) switching off the UV light emitter when a disinfection operational mode is complete, and
(f) enabling a visible light emitter when the disinfection operational mode is complete.

The method may comprise one or more of the following features or steps, considered alone or in combination with one another:
- the method further comprises installing the lighting system within an enclosure, wherein the enclosure is configured to be installed within the fuselage of an aircraft in compliance with relevant airworthiness regulations and is certified as such by the appropriate airworthiness authority;
- the method further comprises installing the lighting system within and interior of a train, boat, bus, or other transportation vehicle;
- the method further comprises installing the lighting system within a lavatory of a hotel or other public place.

### BRIEF DESCRIPTION OF THE DRAWINGS

Figure 1 is a diagram of typical aircraft enclosure including a lighting system, light system module, one or more presence detectors, door locked/unlocked sensor, door open/closed sensor and a controller;
Figure 2 is a diagram of a light system module combining both a visible light emitter with a UV light emitter;
Figure 3 is a diagram of the light system module and its effective electromagnetic range within the enclosure;
Figure 4 is an illustration of a process for determining the safe use of an UV light emitter within an enclosed space.

Like numerals refer to like parts throughout the several views of the drawings.

### DETAILED DESCRIPTION

The following description and drawings are illustrative and are not to be construed as limiting. Numerous specific details are described to provide a thorough understanding of the disclosure. However, in certain instances, well-known or conventional details are not described in order to avoid obscuring the description. References to one or an embodiment in the present disclosure can be, but not necessarily are references to the same embodiment; and, such references mean at least one of the embodiments. If a component is not shown in a drawing then this provides support for a negative limitation in the claims stating that that component is "not" present. However, the above statement is not limiting and in another embodiment, the missing component can be included in a claimed embodiment.

Reference in this specification to "one embodiment," "an embodiment," "a preferred embodiment" or any other phrase mentioning the word "embodiment" means that a particular feature, structure, or characteristic described in connection with the embodiment is included in at least one embodiment of the-disclosure and also means that any particular feature, structure, or characteristic described in connection with one embodiment can be included in any embodiment or can be omitted or excluded from any embodiment. Furthermore, any particular feature, structure, or characteristic described herein may be optional.

The subject matter of embodiments of the present invention is described here with specificity to meet statutory requirements, but this description is not necessarily intended to limit the scope of the claims. The claimed subject matter may be embodied in other ways, may include different elements or steps, and may be used in conjunction with other existing or future technologies. This description should not be interpreted as implying any particular order or arrangement among or between various steps or elements except when the order of individual steps or arrangement of elements is explicitly described.

Although the disclosed embodiments are described with respect to aircraft and other passenger transportation vehicles, they are by no means so limited. Rather, embodiments may be used in other appropriate environments. For example, the lighting system may be installed and used in a lavatory, or other enclosure, in a hotel, stadium, movie theater, or other enclosed public place. The lighting system may be installed and used in a lavatory, or other enclosure, within the interior of a train, boat, bus, or other transportation vehicle. It should be understood that aircraft and passenger transportation vehicles present unique challenges with respect to turn-around time, weight restrictions, airworthiness, and other regulations that are specific to certain industries and may find particular beneficial use of this disclosure.

Aircraft flying in commercial service have to be healthy and safe for passengers, fuel efficient and comply with applicable airworthiness regulations. The airframe manufacturers, and their suppliers, in cooperation with Government airworthiness authorities, such as the Federal Aviation Administration (FAA) in the United States and the European Union Aviation Safety Agency (EASA) have developed procedures, regulations, processes and certification requirements to ensure products that are introduced into the aircraft are safe and meet airworthiness regulations. (Herein, the FAA will be used as the exemplary governmental regulatory agency or administration.) The FAA has certification requirements that must be met in order for a lighting system, for example, to be installed in an aircraft. Such certification requirements include, but are not necessarily limited to, flammability, health and weight/load certification. Meeting such certification requirements could entail for example, but are not limited to, the use of materials that comply with heat, smoke, and toxicity requirements.

Visible light provided by the visible light emitter allows the passenger to see while in the enclosed space, as well as potentially improves the perception of cleanliness and hygiene in the lavatory. In addition to providing visible light lighting systems, there is also a need to neutralize pathogens in an enclosed space or enclosure, for example an aircraft lavatory, between subsequent uses by passengers. UV light is a preferred method of neutralizing pathogens but its use is not safe for human exposure. Humans should not look at or otherwise be exposed directly to UV light in the wavelength that is used for disinfection of pathogens. (Herein, the term "pathogens" is used to include both viral and bacterial pathogens, or any other microbial organism or contaminant. The terms "anti-viral" and "anti-bacterial" are used to refer to neutralizing the effect of such pathogens on living beings.) Accordingly, this disclosure provides both a visible light emitter and a UV light emitter in a single module lighting system, but also incorporates safety features for preventing the UV light emitter from being activated when there is an occupant in the enclosure. The disclosed solution provides an FAA compliant pathogen neutralizing UV light source and visible lighting in a single module.

In one example, the surface disinfection and visible lighting system disclosed is the combination of a light system module containing both a UV light emitter and a visible light emitter, one or more presence detectors, a sensor to detect if the enclosure door is opened or closed, and a sensor to detect if the enclosure door is locked or unlocked, all of which are controlled by a controller. The controller may include a digital electronic circuit with logic gates to manage the UV light emitter, the visible light emitter, the signals received from the one or more presence detectors, the signal received from the door lock/unlock sensor, and the signal received from the door open/closed sensor. By coupling the disinfection function and the visible light function, the disclosed lighting system can provide a clean environment that is free of many harmful bacteria and viruses, provide visible light, and improve the cleanliness perception to the passenger, all while ensuring passenger safety in compliance with airworthiness regulations. The combination of all these features into the same light system allows the whole light system to be in one module and in one place, which saves space, weight, and installation time.

To be effective at safely neutralizing pathogens in compliance with airworthiness regulations and providing visible light within the enclosure, the lighting system includes a light system module which contains a UV light emitter with a "disinfection operational mode." The UV light emitter may emit UVC light within the wavelength of about 200 nm to about 280 nm. Optimally, the UV light emitter emits UVC light sources with a wavelength of between about 250 nm and about 280 nm. The electromagnetic range of the UV light emitter may be limited to the range size of the inside of the enclosure, for example a lavatory.

To ensure a safe operation of the system in presence of passengers, operation of the UV light emitter is limited to use when its disinfection operational mode is activated. The disinfection operational mode can only be activated when no passenger or animal, such as a service dog, is detected within the enclosure. This detection may be via a presence detector, a door sensor, or a door lock/unlock detection sensor. This can ensure that the controller will not activate the UV light emitter unless (a) the enclosure door is closed and (b) when the enclosure door is unlocked (indicating no occupant is present within the enclosure). When the enclosure's door opened/closed sensor detects that the door to the enclosure is open (or is being opened) and/or or when the enclosure's door locked/unlocked sensor detects that the door is locked (indicating that an occupant is present within the enclosure), the UV light emitter will be turned off by the controller. In addition, the visible light emitter (which provides visible spectrum light) of the light system module is enabled when a passenger is inside the enclosure, or while a passenger is entering and exiting the enclosure.

In addition to the light system module, the lighting system includes one or more presence detectors that are designed to detect the presence of passengers or animals within the enclosure and send a signal (e.g., present or not present) to a controller. Such presence detectors may include, but are not necessarily limited to, motion detectors, thermal detectors, detectors that sense weight on the floor, or cameras. The sensors for the door locked/unlocked and door open/closed status may be situated on or adjacent to the enclosure door, or other enclosure access point. When the controller receives signals from the one or more presence detectors, the door locked/unlocked sensor, and/or the door open/closed sensor, the controller directs the light system module to either activate the UV light emitter or enable the visible light emitter accordingly. For example, if an occupant is detected as being present within the enclosure (based on the system detection described), the visible light emitter is activated. If the system detection indicates to the controller that an occupant is not detected as being present within the enclosure, the UV light emitter is activated. The disinfection operation mode allows an automatic surface disinfection when no passengers are present between flights, and also while passengers are boarding or disembarking.

Additionally, when the UV light emitter's disinfection operational mode is complete the controller turns off the UV light emitter, this can enable activation of the visible light emitter within the enclosure. It should also be understood that if the UV light emitter has operated for any appropriate predetermined threshold of time (e.g., a few seconds to a few minutes) and no occupant attempts to enter the enclosure after disinfection has taken place, the controller may turn off the UV light emitter. The visible light emitter may also be turned off, just remaining on standby. This is necessary particularly for instances when the aircraft is between flights or out of service.

In use, the disinfection operational mode neutralizes pathogens within the enclosure, and the visible light operational mode provides visible light within the enclosure. The operational modes are selected based on sensed values from one or more presence detectors that detect the presence of an occupant (passenger or animal) inside the enclosure or lavatory and signals the lighting system controller accordingly. In a specific example, the system for the detection of an occupant includes one or more presence detectors, an enclosure door locked/unlocked sensor, an enclosure door open/closed sensor, and a controller. In a preferred embodiment, the use of one or more presence detectors, as well as the different locations of such presence detectors, allow for a safe use of the UVC light in an enclosure, such as an aircraft lavatory, when passengers and animals are not present.

In a preferred embodiment, The UV light emitter has a wavelength between 200 nm and 280 nm, commonly known as UVC. This spectrum range is not visible to humans therefore the effect of the UV light emitter neutralizing pathogens is not visibly perceptible to the passengers. Additionally, the exposure of UVC light (especially above 250 nm) is considered to be carcinogenic to humans. Thus, a reliable sensor system is needed to detect the presence of passengers or other animals inside the lavatory, or other enclosure, to ensure a safe environment. UVC light with a wavelength comprised between 250 nm and 280 nm has the most pathogen neutralizing effect in the UVC, UVB and UVA range.

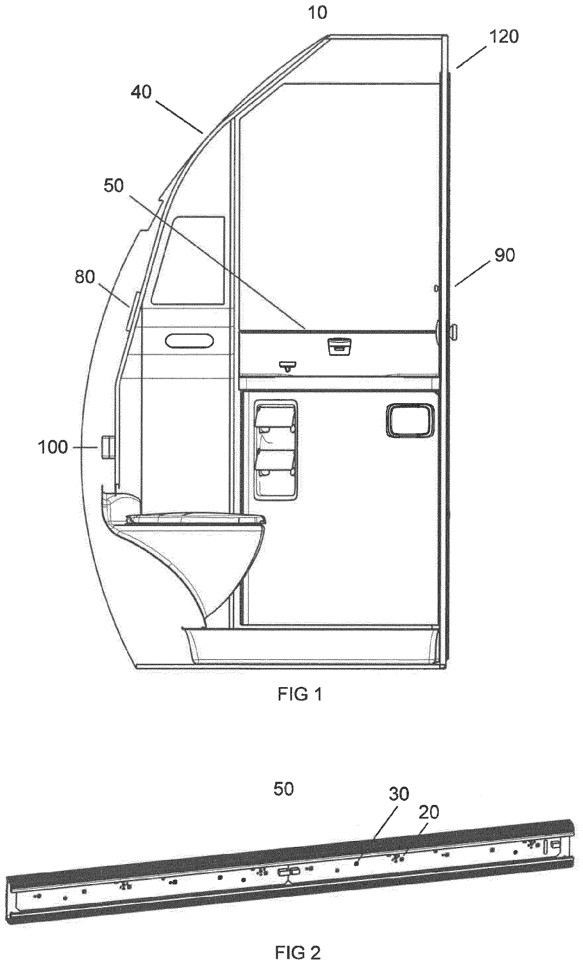

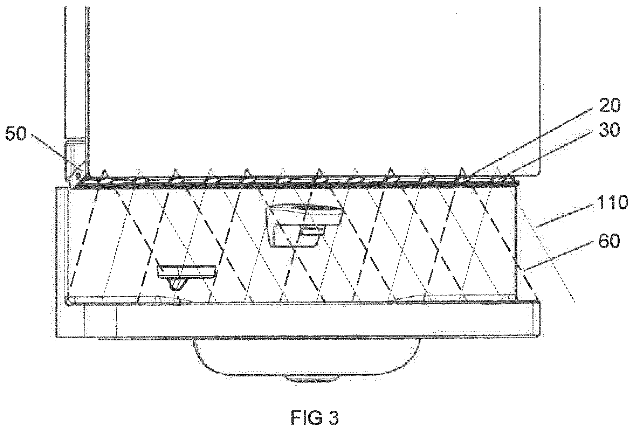

Referring now to the drawings, which are for purposes of illustrating the present invention and not for purposes of limiting the same, FIGS. **1-3** show a lighting system **10,** and method for using the same, that is configured to be installed in an enclosure **40.** FIG **1** shows the lighting system **10** installed in an enclosure **40** wherein an exemplary embodiment of the enclosure **40** is an aircraft lavatory. However, it will be appreciated that the enclosure can be any type of interior enclosure typically found on an aircraft, such as a closet, galley, crew rest, cockpit, overhead stowage bin, or any other enclosure. Further, the enclosure is not limited to enclosures found on aircraft but may include enclosures found on a train, boat, bus or other transportation vehicle and also may include enclosures found in a hotel, stadium, movie theater or other public place.

In a preferred embodiment, the lighting system **10** includes a light system module **50,** such light system module **50** comprising a UV light emitter **20** and a visible light emitter **30,** both contained within the same light system module **50.** The UV light emitter **20** is capable of emitting UV light with a wavelength between 250 nm and 280 nm and is capable of neutralizing pathogens within its electromagnetic range **60.** The visible light emitter **30** is capable of emitting light **110** in the visible spectrum and providing illumination within the enclosure **40.**

In one example, enclosure **40** may incorporate one or more presence detectors **80** configured to detect the presence of occupant, such as a person or animal, within the enclosure **40.** Such presence detectors **80** may consist of, but are not necessarily limited to, motion detectors, thermal detectors, detectors that sense weight on the floor, or cameras.

In one example, the enclosure **40** may incorporate a door locked/unlocked sensor **90** configured to detect whether the enclosure **40** door is locked or unlocked. Additionally or alternatively, the enclosure **40** may incorporate a door open/closed sensor **120** configured to detect whether the enclosure **40** door is open or closed. Additionally or alternatively, the enclosure **40** may incorporate one or more presence detectors **80** to detect the presence of people or animals within the enclosure **40.** In one specific example, the enclosure **40** incorporates at least one of each of the above described detectors or sensors. The detectors and/or sensors detect information that can help determine whether an occupant is present in the enclosure. The detectors and/or sensors send this information to a controller **100.** In a specific example, each detector and/or sensor sends a status signal to the controller **100.**

The system also includes a controller **100** that works in conjunction with the described safety features (e.g., the one or more presence detectors **80,** the door locked/unlocked sensor **90,** and/or the door open/closed sensor **120)** to ensure that enclosure **40** is not occupied by a person or animal before the lighting system's **10** UV light emitter **20** emits UV light within the enclosure **40** and throughout the UV light emitter's **20** electromagnetic range **60.** Further, the controller **100** works in conjunction with the one or more presence detectors **80,** the door locked/unlocked sensor **90,** and/or the door open/closed sensor **120** to enable the visible light emitter **30** to provide visible light **110** to the people or animals within the enclosure **40** when the UV light emitter **20** is deactivated by the controller **100.**

As discussed above, airworthiness authorities, such as the FAA or EASA, have developed procedures, regulations, processes and certification requirements to ensure products that are introduced into aircraft are safe and meet all such airworthiness authorities' applicable requirements and regulations. In a preferred embodiment, the interior enclosure **40** is certified by the appropriate governmental agency, such as the FAA or EASA. Such certification requirements include, but are not necessarily limited to, flammability, health, and weight/load certification. Meeting such certification requirements could entail for example, but are not limited to, the use of materials that comply with heat, smoke, and toxicity requirements.

In a further embodiment, the lighting system **10,** the one or more presence detectors **80,** the door locked/unlocked sensor **90,** the door open/closed sensor **120** and the controller **100** are installed into an enclosure **40** that has previously been certified as airworthy by the relevant airworthiness authority. This installation compliments the previous certification and allows the enclosure **40** to remain certified airworthy by the relevant airworthiness authority

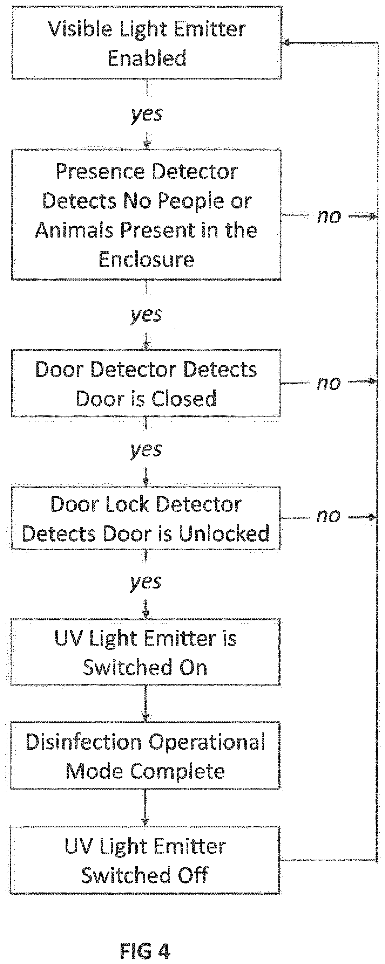

The flow chart of FIG. 4 is intended to illustrate the operational parameters, system architecture and system functionality of the embodiments of the lighting system **10.** In this regard each block in the flow chart may represent, but is not necessarily limited to a function, or step of a function, within the overall system. In some alternative embodiments, the blocks or steps of the flow chart may occur in different orders, may be combined into one block, or may be divided over several blocks.

Such blocks or steps include, but are not necessarily limited to:
(a) detecting there are no people or animals within the enclosure **40,**
(b) detecting the enclosure **40** door is closed,
(c) detecting the enclosure **40** door is unlocked,
(d) neutralizing pathogens within the enclosure **40** by switching on the light system module's **50** UV light emitter **20** when the controller **100** has determined the disinfection operational mode can be activated,
(e) switching off the light system module's **50** UV light emitter **20** when the disinfection operational mode is complete, and
(f) enabling the light system module's **50** visible light emitter **30** when the disinfection operational mode is complete.

In the following, further examples are described to facilitate the understanding of the invention:
Example A. In one example, there is provided a lighting system comprising: a light system module, wherein such light system module contains both UV light emitters and visible light emitters.
Example B. The component of any of the preceding or subsequent examples, wherein the UV light emitter is anti-microbial in nature, anti-viral in nature, or both.
Example C. The component of any of the preceding or subsequent examples, wherein the lighting system is used or installed within an enclosure.
Example D. The component of any of the preceding or subsequent examples, further comprising one or more of: one or more presence detectors that detect the presence of people or animals within an enclosure and/or within the electromagnetic range of the UV light emitter; a door locked/unlocked sensor; a door open/closed sensor; or any combination thereof.
Example E. The component of any of the preceding or subsequent examples, further comprising a controller that manages the light system module, the one or more presence detectors, the door locked/unlocked sensor, and/or the door open/closed sensor.
Example F. The component of any of the preceding or subsequent examples, wherein the lighting system is certified by the relevant airworthiness authority to be compliant with applicable airworthiness regulations.
Example G. The component of any of the preceding or subsequent examples, wherein the lighting system is configured to be installed within the fuselage of an aircraft.
Example H. The component of any of the preceding or subsequent examples, wherein the lighting system is configured to be installed within an interior of a train, boat, bus, or other transportation vehicle.
Example 1. The component of any of the preceding or subsequent examples, wherein the lighting system is configured to be installed in a lavatory of a hotel, a stadium, a movie theater, or other enclosed public place.
Example J. In a further embodiment, there may be provided a lighting system for installation in an enclosure to be disinfected, the lighting system comprising: a light system module that has at least one UV light emitter and at least one visible light emitter, wherein the at least one UV light emitter is anti-microbial in nature, anti-viral in nature, or both, wherein the visible light emitter emits light in the visible spectrum; one or more detectors or sensors, the one or more detectors or sensors comprising (a) a presence detector that detects the presence of people or animals within the enclosure and/or within an electromagnetic range of the at least one UV light emitter, (b) a door locked/unlocked sensor, (c) a door open/closed sensor, or (d) any combination thereof; and a controller that manages the light system module and the one or more detectors or sensors.
Example K. The component of any of the preceding or subsequent examples, wherein the one or more detectors or sensors are configured to detect the presence or absence of an occupant in the enclosure and send detected information to the controller, wherein the controller can activate the at least one UV light emitter if the detected information relays absence of an occupant, wherein the controller can activate the at least one visible light emitter if the detected information relays presence of an occupant.
Example L. There may also be provided a method for using a lighting system to neutralize pathogens and provide visible light within the interior of an enclosure, the method comprising: (a) detecting that there are no people or animals within the enclosure, (b) detecting that the enclosure door is closed, (c) detecting that the enclosure door is unlocked, (d) neutralizing pathogens within the enclosure by switching on a UV light emitter when a controller has determined the disinfection operational mode can be activated, (e) switching off the UV light emitter when a disinfection operational mode is complete, and (f) enabling a visible light emitter when the disinfection operational mode is complete.
Example M. The component of any of the preceding or subsequent examples, further comprising installing the lighting system within an enclosure, wherein the enclosure is configured to be installed within the fuselage of an aircraft in compliance with relevant airworthiness regulations and is certified as such by the appropriate airworthiness authority.
Example N. The component of any of the preceding or subsequent examples, further comprising installing the lighting system within and interior of a train, boat, bus, or other transportation vehicle.
Example O. The component of any of the preceding or subsequent examples, further comprising installing the lighting system within a lavatory of a hotel or other public place.

The above-detailed description of embodiments of the disclosure is not intended to be exhaustive or to limit the teachings to the precise form disclosed above. While specific embodiments of and examples for the disclosure are described above for illustrative purposes, various equivalent modifications are possible within the scope of the disclosure, as those skilled in the relevant art will recognize. Further, any specific numbers noted herein are only examples and are not limiting: alternative implementations may employ differing values, measurements or ranges. Any patents and applications and other references noted above, including any that may be listed in accompanying filing papers, are incorporated herein by reference in their entirety.

Different arrangements of the components depicted in the drawings or described above, as well as components and steps not shown or described are possible. Similarly, some features and sub-combinations are useful and may be employed without reference to other features and sub-combinations. Embodiments of the invention have been described for illustrative and not restrictive purposes, and alternative embodiments will become apparent to readers of this patent. Accordingly, the present invention is not limited to the embodiments described above or depicted in the drawings, and various embodiments and modifications may be made without departing from the scope of the claims below.

## Claims

1. A lighting system (10) comprising:
a light system module (50), wherein such light system module contains both UV light emitters and visible light emitters (20, 30).

2. The lighting system (10) of claim 1, wherein the UV light emitter (20) is anti-microbial in nature, anti-viral in nature, or both.

3. The lighting system (10) of claim 1 or 2, wherein the lighting system is used or installed within an enclosure (40).

4. The lighting system (10) of any one of claims 1 to 3, further comprising one or more of:
one or more presence detectors (80) that detect the presence of people or animals within an enclosure (40) and/or within the electromagnetic range of the UV light emitter (20);
a door locked/unlocked sensor (90);
a door open/closed sensor (120);
or any combination thereof.

5. The lighting system (10) of claim 4, further comprising a controller (100) that manages the light system module (50), the one or more presence detectors (80), the door locked/unlocked sensor (90), and/or the door open/closed sensor (120).

6. The lighting system (10) of any one of claims 1 to 5, wherein the lighting system is certified by the relevant airworthiness authority to be compliant with applicable airworthiness regulations.

7. The lighting system (10) of any one of claims 1 to 6, wherein the lighting system is configured to be installed within the fuselage of an aircraft.

8. The lighting system (10) of any one of claims 1 to 6, wherein the lighting system is configured to be installed within an interior of a train, boat, bus, or other transportation vehicle.

9. The lighting system (10) of any one of claims 1 to 6, wherein the lighting system is configured to be installed in a lavatory of a hotel, a stadium, a movie theater, or other enclosed public place.

10. A lighting system (10) for installation in an enclosure (40) to be disinfected, the lighting system comprising:
a light system module (50) that has at least one UV light emitter (20) and at least one visible light emitter (30), wherein the at least one UV light emitter is anti-microbial in nature, anti-viral in nature, or both, wherein the visible light emitter emits light in the visible spectrum;
one or more detectors (80) or sensors, the one or more detectors or sensors comprising (a) a presence detector that detects the presence of people or animals within the enclosure and/or within an electromagnetic range of the at least one UV light emitter, (b) a door locked/unlocked sensor (90), (c) a door open/closed sensor (120), or (d) any combination thereof; and
a controller (100) that manages the light system module and the one or more detectors or sensors.

11. The lighting system (10) of any one of claims 1 to 10, wherein the one or more detectors or sensors are configured to detect the presence or absence of an occupant in the enclosure and send detected information to the controller, wherein the controller can activate the at least one UV light emitter if the detected information relays absence of an occupant, wherein the controller can activate the at least one visible light emitter if the detected information relays presence of an occupant.

12. A method for using a lighting system (10) to neutralize pathogens and provide visible light within the interior of an enclosure, the method comprising:
(a) detecting that there are no people or animals within the enclosure,
(b) detecting that the enclosure door is closed,
(c) detecting that the enclosure door is unlocked,
(d) neutralizing pathogens within the enclosure by switching on a UV light emitter when a controller has determined the disinfection operational mode can be activated,
(e) switching off the UV light emitter when a disinfection operational mode is complete, and
(f) enabling a visible light emitter when the disinfection operational mode is complete.

13. The method of claim 12, further comprising installing the lighting system (10) within an enclosure, wherein the enclosure is configured to be installed within the fuselage of an aircraft in compliance with relevant airworthiness regulations and is certified as such by the appropriate airworthiness authority.

14. The method of claim 12, further comprising installing the lighting system (10) within and interior of a train, boat, bus, or other transportation vehicle.

15. The method of claim 12, further comprising installing the lighting system (10) within a lavatory of a hotel or other public place.
